# EUROPEAN PATENT SPECIFICATION

(11) **EP 0 798 398 B1**
(45) Date of publication and mention of the grant of the patent: **09.07.2003**
(21) Application number: 97302092.8
(22) Date of filing: 26.03.1997
(51) Int. Cl.: C23C 8/38, A61B 17/06, A61M 5/32

(54) **Process for passivating surgical needles**
Verfahren zur Passivierung von chirurgischen Nadeln
Procédé de passivation d'aiguilles chirurgicales

(30) Priority: 27.03.1996 US 622215
(43) Date of publication of application: 01.10.1997
(73) Proprietor: ETHICON, INC., Somerville, NJ 08876 (US)
(72) Inventor: Vetrecin, Robert, Stewartsville, NJ 08886 (US); Hersey, Bruce, San Angelo, TX 76901 (US)
(74) Representative: Fisher, Adrian John

(56) References cited:
- US-A- 5 062 900
- US-A- 5 384 167
- PATENT ABSTRACTS OF JAPAN vol. 008, no. 222 (C-246), 9 October 1984 & JP 59 105837 A (MITSUBISHI KASEI KOGYO KK), 19 June 1984,
- SCHUEGRAF K.K.: 'Handbook of Thin-Film Deposition Processes and Techniques', 1988, NOYES PUBLICATION, PARK RIDGE, NJ, USA pages 396 - 408

## Description

### TECHNICAL FIELD

The field of art to which this invention pertains is surgical needles, more specifically a method of passivating surgical needles.

### BACKGROUND OF THE INVENTION

Surgical needles are typically manufactured from various grades of metal steel alloys which will not corrode when the needles are exposed to ambient environmental conditions after manufacture and prior to, and during, use. The metal alloys typically may include the 300 and 400 series stainless steels and other conventional alloys such as 420, 420F, 455, and the like. In addition, martensitic stainless steel alloys containing nickel and titanium are useful such as those disclosed in U.S. Patent 5,000,912. Surgical needles and processes for manufacturing surgical needles are disclosed in commonly assigned, co-pending U.S Patent Application Serial Nos. 08/405,554 and 08/429,446. In order to render a surgical needle made from such metal alloys corrosion resistant, it is known that the exterior surface of the needle must be passivated.

The term "passivation" is conventionally defined to mean a process for treating a metal surface to provide a uniform thin oxide layer on the surface. This oxide layer protects the underlying metal surface from corrosion by acting as a gas-impermeable surface. There are a number of known conventional processes for passivating stainless steel alloys and other conventional metal alloys including various chemical and electrochemical processes.

Surgical needles are presently passivated using such conventional passivation processes. The conventional passivation processes which are most typically used are chemical passivation processes and electropolishing passivation processes. The chemical passivation processes utilize various mixtures of acids and aqueous salts to oxidize the surface of a surgical needle, thereby producing an oxidized layer and passivating the needle. For example, a typically used chemical passivation mixture will contain sulfuric acid and potassium dichromate in an aqueous bath, or HNO₃ in an aqueous bath. The aqueous bath is preferably maintained at room temperature although other temperatures may be utilized. Another type of passivation process which is often used is, as previously mentioned, an electrochemical passivation process. In an electrochemical passivation process needles are placed in a chemical bath and an electric current is passed through the bath. Typically utilized in such a process are voltages in the range of about 3V (volts) to about 40V (volts) and high amperages in the range of about 5A (amps) to about 175A (amps).

Electrochemical treatment of a metallic material with a low-temperature plasma using the metallic material as cathode has been used to improve the corrosion resistance of the material by removing impurities from the metal surface (US-A-5,062,900). Electrochemical treatment of a metal surface with plasma has also been used to etch the metal surface and to inject carbon, sufur and fluorine atoms into the surface (US-A-5,384,167). Electrochemical treatment of a metallic substrate under an atmosphere comprising an oxidative gas using the metallic substrate as cathode has been used to coat the metallic substrate with an oxide layer (JP-59,105,837).

Although the existing electrochemical and chemical passivation processes produce needles having adequately passivated surfaces, there are certain disadvantages associated with the use of these processes. The processes require the use of chemical baths generating both chemical fumes and hazardous waste. The chemical baths have a limited lifetime and have to be disposed of at considerable expense. In addition, the use of the types of chemicals required for chemical or electrochemical passivation baths has with it attendant safety hazards which must be constantly monitored. Also, the electrochemical process uses relatively high voltages and amperages and, once again, considerable safety precautions must be taken to protect operators.

Accordingly there is a need in this art for a novel method of passivating the surfaces of metal alloy surgical needles and surgical instruments without the use of chemical or electrochemical processes.

### DISCLOSURE OF THE INVENTION

It is an object of the present invention to provide a process for passivating the surfaces of metal alloy surgical needles and surgical instruments without the use of chemical baths.

It is yet a further object of the present invention to provide a method of passivating the surfaces of surgical needles and surgical instruments without the use of an electrochemical bath.

It is still yet a further object of the present invention to provide a method for passivating the surfaces of a metal alloy surgical needle or surgical instrument without the need for passing an electrical current through the needle or instrument.

Therefore, a process for passivating a metal alloy surgical needle or surgical instrument is disclosed. The metal surgical instrument or surgical needle has an exterior surface. The exterior surface of the metal surgical needle or surgical instrument is exposed to a gaseous plasma for a sufficient period of time at a sufficient temperature to effectively passivate the exterior surface of the needle or instrument. The needle or surgical instrument may also have one or more interior surfaces which may also be passivated.

The novel method of passivation of metal alloy surgical needles or surgical instruments of the present invention has many advantages. Environmental hazards associated with the use of chemical baths and electrochemical baths are eliminated since these chemical baths are not needed. In addition, the gaseous plasma which is used to passivate the exterior surface of the surgical instruments and the surgical needles is recaptured and recycled after each process. Yet another advantage of the present invention is that the need for passing an electric current through the needles and the attendant safety hazards are eliminated.

The foregoing and other features and advantages of the present invention will become more apparent from the following description and accompanying drawings.

### BRIEF DESCRIPTION OF THE DRAWINGS

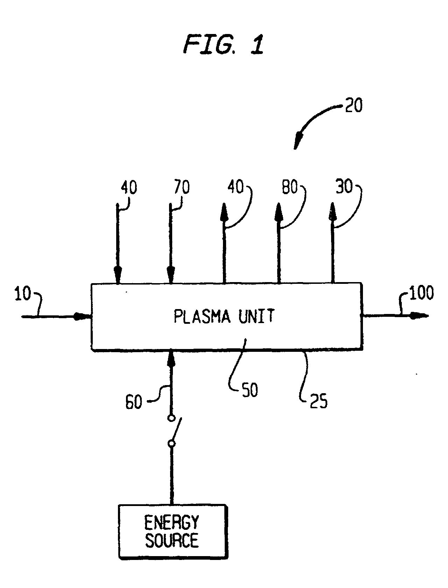

FIG. 1 illustrates a flow diagram for a passivation process of the present invention.

### BEST MODE FOR CARRYING OUT THE INVENTION

The gaseous mixtures which can be used for the plasmas of the present invention consist essentially of oxygen, helium and carbon tetraflouride. Sufficient amounts of each component will be utilized to provide an effective plasma mixture. Typically, about 50% to about 99% of oxygen will be used in such a mixture, more typically about 60% to about 99%, and preferably about 70% to about 99%. The typical amounts of helium which will be used in such a mixture will typically be about 1% to about 50%, more typically about 10% to about 40%, and preferably about 10% to about 30%. The amounts of carbon tetraflouride which will be utilized in a preferred mixture will typically be about 1% to about 40%, more typically about 15% to about 35%, and preferably about 10% to about 30%. The percentages used herein are percentages by weight.

A conventional plasma treatment process unit is typically used in the processes of the present invention. The plasma unit will typically have a volumetric chamber which is capable of withstanding both pressure and vacuum. Mounted in the chamber will be at least one electrode. A preferred unit is a Gasonics® plasma unit manufactured by Gasonics/IPL, San Jose, California, however, any conventional or equivalent gas plasma unit may be utilized. A gaseous mixture is typically brought up to a plasma state in these units by exposing the gas to sufficient electromagnetic energy such as radio frequency electromagnetic waves, microwaves, etc., to effectively induce a plasma state. However, other means of exciting the gas into a plasma state may be utilized including direct current, laser energy, equivalents thereof and the like. If desired, the needles or surgical instruments can be exposed to a conventional plasma torch.

Sufficient electromagnetic energy will be applied to the gas to effectively produce a plasma condition. Typically the amount of energy utilized will be about 250W (0.12 watts/M²) to about 2500W (1.2 watts/M²), more typically to about 300W (.014 watts/M²) to about 1000W (0.46 watts/M²), and preferably about 400W (0.18 watts/M²) to about 900W (0.42 watts/M²). Of course those skilled in the art will appreciate that the amount of energy utilized will vary in accordance with the process parameters including gas flow, gas type, electrode area, and vacuum, etc., as well as the type, size, condition and configuration of the plasma unit.

The gas will flow into the plasma treatment process unit at a sufficient volumetric flow rate to effectively produce a plasma. The chamber of the treatment unit will have a sufficient volume to effectively contain the needles or instruments being treated. These parameters will vary in accordance with the particular parameters of the process and are readily determined by those skilled in the art.

A typical flow diagram for a passivation process of the present invention is illustrated in FIG. 1, although those skilled in the art will appreciate that various steps may be eliminated or added to the processes of the present invention. As illustrated in FIG. 1, the initial step of such a process is to load needles or surgical instruments 10 into a chamber 25 of a plasma treatment device 20. Next, a sufficient vacuum 30 is pulled on the chamber 25 to effectively evacuate the chamber of air and produce a sufficient vacuum to introduce the plasma. Typically, the vacuum may be about 33 Nm⁻² to about 133 Nm⁻² (about 0.25 to about 1 Torr), more typically about 33 Nm⁻² to about 100 Nm⁻² (about 0.25 to about 0.75 Torr), and preferably about 40 Nm⁻² to about 67 Nm⁻² (about 0.3 to about 0.5 Torr), however this will vary with the type and configuration of plasma unit utilized. Then, the chamber 25 is filled with a gas mixture 40 of choice so that there is a sufficient amount of gas 40 present in the chamber 25 to effectively form the gas plasma 50. Typically the amount of gas flow 40 utilized may be about 50 to about 500 cc/min, more typically about 100, to about 500 cc/min, and preferable about 200 to about 500 cc/min, however this may vary depending upon the type and configuration of the plasma unit utilized. Next the power is switched on to the gas plasma unit thereby allowing the energy source 60 to be activated and form the plasma 50, and the needles or surgical instruments 10 are exposed to the gas plasma 50 for a sufficient period of-time to effectively produce an effective passivation coating on the outer surface of the needles or surgical instruments 10. Typically, the plasma cycle time may be about 10 to about 60 minutes, more typically about 20 to about 40 minutes, and preferably about 30 to about 45 minutes, however, this will vary depending upon the process cycle, process parameters, and plasma unit type and configuration utilized. Next, the gas 40 is removed through vent 80 and the chamber 25 is back-filled with an inert gas 70, such as nitrogen, and the chamber 25 maintained at a sufficient pressure for a sufficient amount of time to effectively cool down the needles or instruments. For example, the needles or instruments 10 may be held in the cool down phase for about 3 to about 10 minutes, more typically about 3 to about 7 minutes, and preferably about 3 to about 5 minutes. The inert gas pressure may be, for example, about 7 Nm⁻² to about 133 Nm⁻² (about 0.05 Torr to about 1.0 Torr). Finally, the passivated needles or instruments 10 are removed from the chamber 25 of the gas plasma unit 20. The gas 40 is removed through vent 80 and may be recycled for use in the process.

The oxide layers produced by the plasma treatment passivation process of the present invention will be sufficiently thick to effectively protect the underlying metal from corrosion. Typically, the oxide layers will be of conventional thicknesses, e.g., monatomic.

The surgical needles which can be passivated using the process of the present invention include any conventional surgical needle having a piercing point, sharp or blunt, and a suture mounting end. The suture mounting end may have a channel or a blind drilled hole for receiving sutures. The surgical instruments which may be passivated using the process of the present invention include conventional instruments, and parts thereof, such as needle graspers, scissors, forceps, scalpels, catheters, cutting instruments, clamps, saws, and the like. The term surgical instrument as used herein is defined to include a metallic part of a surgical instrument. The needles and surgical instruments will typically have external surfaces which will be passivated using the process of the present invention, however, they may also have interior surfaces which may be passivated as well.

### Comparative Example

Approximately 3000 surgical needles made from 420 stainless steel metal alloy were placed into the chamber of a Gasonics® Plasma Unit. The chamber had a volume of about 0.113 m³ (4 ft³). The door to the unit was sealed and the chamber was evacuated under a vacuum of about 20 Nm⁻² (0.15 Torr) for about one minute to purge volatiles and contaminants. The chamber was next back-filled with a mixture of 50 cc/minute of oxygen and 50 cc/minute of Helium to a pressure of about 40 Nm⁻² to about 67 Nm⁻² (about 0.3 to about 0.5 Torr). The gaseous mixture was maintained in the chamber prior to switching power on for about 2 minutes. Next, power was switched on to the unit and the needles were exposed to the resulting gaseous plasma which was maintained at a power level of about 500 watts for about 30 minutes. Next, the power was switched off and the gaseous mixture was evacuated from the chamber. Then, the chamber was filled with nitrogen and held at a pressure of about 133 Nm⁻² (1.0 Torr) for approximately 3-5 minutes until the needles were cool enough to be handled. The needles were then removed from the chamber of the gas plasma unit. Upon inspection, the surfaces of the needles were passivated and the needles had a uniform oxide coating of conventional thickness.

The process of the present invention for passivating the surfaces of metal alloy surgical needles and surgical instruments has many advantages. Surprisingly and unexpectedly, it is now possible to passivate the surfaces of metal alloy surgical needles and surgical instruments in a controlled process which does not utilize chemical or electrochemical baths and which does not generate the associated fumes, emissions and hazardous waste streams. Using the plasma process of the present invention, it is possible to recycle the gaseous plasma agents. In addition, the use of high amperage electrical currents in order to electrically passivate needles is eliminated. Yet another advantage of the present invention is that needles which are processed in the gas plasma processes of the present invention are not subjected to the metal removal which is a characteristic disadvantage of the chemical or electrochemical processes. Still yet another advantage of the process of the present invention is that it is significantly more economical and cost effective than the prior art conventional passivation processes. The process of the present invention also eliminates the safety concerns associated with the conventional passivation processes.

Although this invention has been shown and described with respect to detailed embodiments thereof, it will be understood by those skilled in the art that various changes in form and detail thereof may be made without departing from the scope of the claimed invention.

## Claims

1. A method of passivating the surface of a metallic surgical needle, the method comprising:
exposing a metallic surgical needle having an exterior surface to a gaseous plasma for a sufficient period of time at a sufficient temperature to effectively passivate the exterior surface of the needle, wherein the plasma mixture consists essentially of oxygen and 1 wt.% to 50 wt.% of helium and 1 wt.% to 40 wt.% of carbon tetrafluoride, and wherein the gaseous plasma is excited by an electromagnetic energy source.

2. A method of passivating the surface of a metallic surgical instrument, the method comprising:
exposing a metallic surgical instrument having an exterior surface to a gaseous plasma for a sufficient period of time at a sufficient temperature to effectively passivate the exterior surface of the instrument, wherein the plasma mixture consists essentially of oxygen and 1 wt.% to 50 wt.% of helium and 1 wt.% to 40 wt.% of carbon tetrafluoride, and wherein the gaseous plasma is excited by an electromagnetic energy source.

3. The method of claim 1, wherein the metallic needle comprises an alloy selected from the group consisting of T-420 stainless steel, T-420F stainless steel, T-455 stainless steel, and titanium nickel martensitic stainless steel alloy.

4. The method of claim 2, wherein the metallic instrument comprises an alloy selected from the group consisting of T-420 stainless steel, T-420F stainless steel, T-455 10 stainless steel, and titanium nickel martensitic stainless steel alloy.

5. The method of any preceding claim wherein the electromagnetic energy is selected from the group consisting of Radio Frequency, Microwave and Laser energy.

6. The method of claim 1 wherein the needle additionally comprises an interior surface and the interior surface is also passivated.

7. The method of claim 2 wherein the instrument additionally comprises an interior surface and the interior surface is also passivated.

## Patentansprüche

1. Verfahren zum Passivieren der Oberfläche einer metallischen, chirurgischen Nadel, wobei das Verfahren umfasst:
Eine metallische, chirurgische Nadel wird mit einer äußeren Oberfläche einem gasförmigen Plasma für eine hinreichende Zeitdauer bei einer hinreichenden Temperatur ausgesetzt, um die äußere Oberfläche der Nadel wirksam zu passivieren, wobei das Plasmagemisch im wesentlichen aus Sauerstoff und 1 Gewichtsprozent bis 50 Gewichtsprozent Helium und 1 Gewichtsprozent bis 40 Gewichtsprozent Kohlentetrafluorid besteht, und wobei das gasförmige Plasma durch eine elektromagnetische Energiequelle angeregt wird.

2. Verfahren zum Passivieren der Oberfläche eines metallischen chirurgischen Instrumentes, wobei das Verfahren umfasst:
Ein metallisches, chirurgisches Instrument wird mit einer äußeren Oberfläche einem gasförmigen Plasma für eine hinreichende Zeitdauer bei einer hinreichenden Temperatur ausgesetzt, um die äußere Oberfläche des Instruments wirksam zu passivieren, wobei das Plasmagemisch im wesentlichen aus Sauerstoff und 1 Gewichtsprozent bis 50 Gewichtsprozent Helium und 1 Gewichtsprozent bis 40 Gewichtsprozent Kohlentetrafluorid besteht, und wobei das gasförmige Plasma durch eine elektromagnetische Energiequelle angeregt wird.

3. Verfahren nach Anspruch 1, wobei die metallische Nadel aus einer Legierung, ausgewählt aus der Gruppe bestehend aus T-420 nicht rostendem Stahl, T-420F nicht rostendem Stahl, T-455 nicht rostendem Stahl und einer martensitischen Edelstahllegierung aus Titan und Nickel besteht.

4. Verfahren nach Anspruch 2, wobei das metallische Instrument aus einer Legierung, ausgewählt aus der Gruppe bestehend aus T-420 nicht rostendem Stahl, T-420F nicht rostendem Stahl, T-455 nicht rostendem Stahl und einer martensitischen Edelstahllegierung aus Titan und Nickel besteht.

5. Verfahren nach einem vorhergehenden Anspruch, wobei die elektromagnetische Energie ausgewählt ist aus der Gruppe bestehend aus Radiofrequenz, Mikrowellen und Laserenergie.

6. Verfahren nach Anspruch 1, wobei die Nadel zusätzlich eine Innenfläche umfasst und die Innenfläche ebenfalls passiviert wird.

7. Verfahren nach Anspruch 2, wobei das Instrument zusätzlich eine Innenfläche umfasst und die Innenfläche ebenfalls passiviert wird.

## Revendications

1. Procédé de passivation de la surface d'une aiguille chirurgicale métallique, le procédé comprenant les étapes consistant à :
- exposer une aiguille chirurgicale métallique ayant une surface extérieure à un plasma gazeux pendant une période de temps suffisante à une température suffisante pour passiver effectivement la surface extérieure de l'aiguille,
- dans lequel le mélange de plasma est constitué essentiellement d'oxygène avec de 1 % à 50 % en poids d'hélium et de 1 % à 40 % en poids de tétrafluorure de carbone, et
- dans lequel le plasma gazeux est excité par une source d'énergie électromagnétique.

2. Procédé de passivation de la surface d'un instrument chirurgical métallique, le procédé comprenant les étapes consistant à :
- exposer un instrument chirurgical métallique ayant une surface extérieure à un plasma gazeux pendant une période de temps suffisante à une température suffisante pour passiver effectivement la surface extérieure de l'instrument,
- dans lequel le mélange de plasma est constitué essentiellement d'oxygène avec de 1 % à 50 % en poids d'hélium et de 1 % à 40 % en poids de tétrafluorure de carbone, et
- dans lequel le plasma gazeux est excité par une source d'énergie électromagnétique.

3. Procédé selon la revendication 1, dans lequel l'aiguille métallique comprend un alliage sélectionné parmi le groupe composé d'un acier inoxydable T-420, d'un acier inoxydable T-420F, d'un acier inoxydable T-455 et d'un alliage d'acier inoxydable martensitique de nickel et de titane.

4. Procédé selon la revendication 2, dans lequel l'instrument métallique comprend un alliage sélectionné parmi le groupe composé d'un acier inoxydable T-420, d'un acier inoxydable T-420F, d'un acier inoxydable T-455 et d'un alliage d'acier inoxydable martensitique de nickel et de titane.

5. Procédé selon l'une quelconque des revendications précédentes, dans lequel l'énergie électromagnétique est sélectionnée parmi le groupe composé de fréquences radio, de micro-ondes et d'énergie laser.

6. Procédé selon la revendication 1, dans lequel l'aiguille comprend, de façon additionnelle, une surface intérieure et la surface intérieure est également passivée.

7. Procédé selon la revendication 2, dans lequel l'instrument comprend, de façon additionnelle, une surface intérieure et la surface intérieure est également passivée.
